# EUROPEAN PATENT APPLICATION

(11) **EP 4 306 482 A1**
(43) Date of publication of application: **17.01.2024**
(21) Application number: 23184848.2
(22) Date of filing: 11.07.2023
(51) Int. Cl.: C01B 32/05, A61L 27/40, B01J 6/00

(54) **METHOD FOR THE PREPARATION OF A MATERIAL**

(30) Priority: 13.07.2022 IT 202200014722
(71) Applicant: Nite Technology OÜ, 10141 Tallinn (EE)
(72) Inventor: CAPODICASA, Alessandro, 96100 Siracusa (IT)
(74) Representative: Braidotti, Andrea

(57) **Abstract**

Method for the preparation of a material comprising, preferably in operative sequence, the following steps:
- a step of making at least one raw material available comprising at least one vegetable material and at least one catalyst,
- a supercritical solvothermal carbonization step, wherein said raw material is subjected to a carbonization process in a supercritical temperature and pressure range in order to obtain a carbonized byproduct;
- a de-agglomeration step, wherein the carbonized byproduct is subjected to de-agglomeration, preferably by ultrasonication, in order to obtain a de-agglomerated byproduct;
- a crushing step, during which the de-agglomerated byproduct is subjected to a crushing process, preferably by the action of an electromagnetic field, more preferably at a high frequency;
a subcritical dehydration step, during which the de-agglomerated byproduct is subjected to a dehydration process under subcritical conditions in order to obtain the material according to the invention.

## Description

### TECHNICAL FIELD

This invention concerns a method for the preparation of a material, in particular a material having antibacterial/antiviral properties, for instance for the use in surgical and/or medical devices and/or in the veterinary sector.

### BACKGROUND ART

Following the pandemic relating to the Sars-Cov-2 virus, the demand for chemical products to be allocated to disinfection, hygiene of the person or to cleaning surfaces has grown significantly in the market.

In order to meet the needs of this industry, different materials with antibacterial and/or antiviral properties are known; however, these are expensive and/or complicated to produce.

The need is further known, at an industrial level, to create protective devices that present excellent antimicrobial and/or antiviral properties in order to limit the spread of bacteria, viruses, mold or other microorganisms.

In particular, the possibility of creating products with antibacterial and/or antiviral properties through the use of chemical synthetic compounds, such as normal antibiotics, or antiviral compounds, is known. However, these products are not fully satisfactory because, although they are effective in their performance, they are expensive and complicated to produce. This situation was further worsened by the deficiencies in the production chain that occurred following the pandemic of Covid 19, with scarcity and problems in the supply of many reagents and chemicals.

### PURPOSES OF THE INVENTION

A purpose of the invention is to propose a method that allows to create material with antibacterial and/or antiviral properties that allows to overcome the problems of known methods for the preparation of these materials.

Another purpose of the invention is to propose a method that provides to start from raw materials of vegetable origin.

Another purpose of the invention is to propose a method that can be implemented in a simple and quick way and with low costs.

Another purpose of the invention is to propose a method that is an alternative and/or improvement compared to those present in the state of the art.

Another purpose of the invention is to propose a method that exploits the properties of innovative catalysts.

Another purpose of the invention is to propose a method that allows to create a nanometric material.

Another purpose of the invention is to propose a method that allows to create a material with which it is possible to create medical devices in a simple and quick way.

Another purpose of the invention is to propose a method that allows to exploit the properties of vegetable molecules.

Another purpose of the invention is to offer a method that uses mainly products easily found on the market.

Another purpose of the invention is to propose a medical device that uses a material according to the invention.

### SUMMARY OF THE INVENTION

All the purposes mentioned here, considered both individually and in any combination thereof, and others that will result from the description that follows are achieved, according to the invention, with a method according to claim 1, and with a material according to claim 10.

### DESCRIPTION OF THE FIGURES

The present invention is further clarified below in some of its preferred forms of practical realization reported for purely exemplary purposes and not limiting with reference to the attached tables of drawings, wherein:

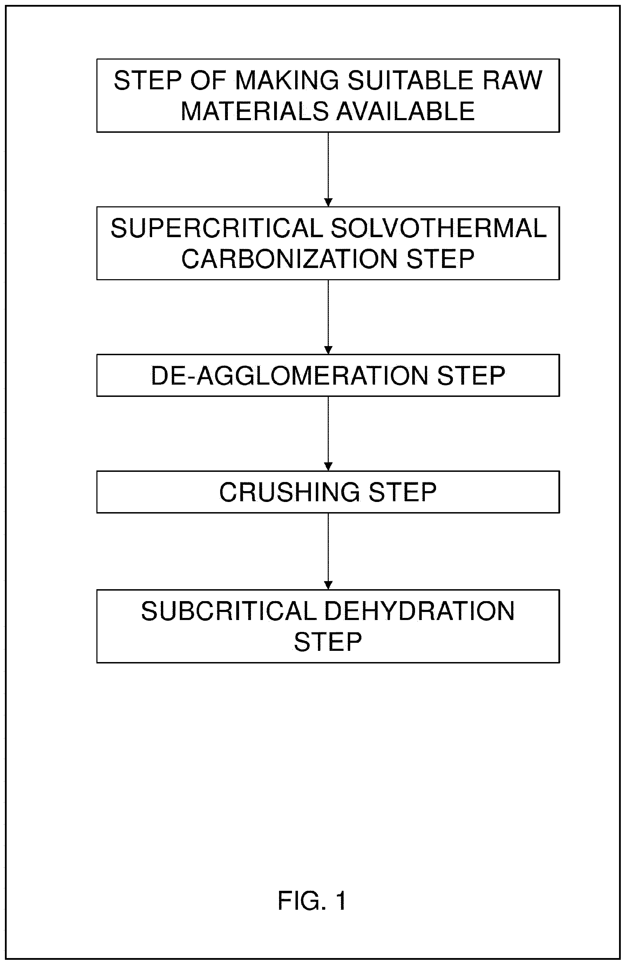

Figure 1 shows in a schematic view the steps of the method according to the invention,
Figure 2a shows an image of the carbonized byproduct made through an electronic scan microscope,
Figure 2b shows an image of the de-agglomerated byproduct made through an electronic scan microscope,
Figure 2c shows an image of the crushed byproduct made through an electronic scan microscope,
Figure 2d shows an image of the dehydrated byproduct made by means of an electronic scan microscope,
Figure 3 shows a spectrum in the Raman spectroscopy of the sample relating to example 1,
Figure 4 shows a spectrum in the Raman spectroscopy of the sample relating to example 2.

### DEFINITIONS

Within the scope of this invention, the following definitions are given:
- with the wording "Solvothermal Carbonization" is meant a chemical process wherein the organic material is transformed into carbon by using solvents and high temperatures. The process usually involves the thermal decomposition of the organic material in a closed and oxygen-free environment, which avoids the complete combustion of the material;
- "Supercritical solvothermal carbonization" means a solvothermal carbonization performed in supercritical conditions, that is, a chemical process that involves the use of supercritical solvents to convert organic materials into carbon. During this process, the organic material is treated in a high pressure and temperature reactor, wherein the solvent is brought to the supercritical state. During the thermal decomposition, a series of chemical reactions occurs, including dehydration, decarboxylation and polymerization. These reactions transform the organic material into solid carbon, eliminating volatile elements such as water, carbon dioxide and other gaseous compounds. The supercritical solvent, acting as an heat transfer medium and facilitating chemical reactions, contributes to the formation of a porous carbon structure with high adsorption characteristics.
- with the wording "subcritic dehydration" is meant a process used to remove water from solid or liquid materials by the application of heat and the creation of a high pressure environment. Unlike conventional dehydration, which takes place at very high temperatures, subcritic dehydration allows to maintain the temperature below the boiling point of water.

- with the wording "nano-cluster" is generally meant a structure consisting of a small number of atoms or molecules that aggregate together to form a particle of nanometric dimensions. Nano clusters are considered interfaces between atomic material and macroscopic material. The size of the nano clusters can vary from few atoms to several hundred atoms. They can be composed of different types of atoms or molecules, and their chemical composition can influence the properties and behavior of the clusters. The shape of the nano clusters can vary from spherical to multifaceted, depending on the arrangement of the entire atoms and forces involved.
- " Filtering medium " wording means a component used in filtering systems to remove unwanted and contaminating particles. The filtering medium for air purification consists of a layer of porous material that acts as a physical barrier for particles in suspension. This material can be made with different types of synthetic or natural fibers, such as fiberglass, cotton, polypropylene or polyester. (American Society of Heating, Refrigarating and Air-Coniction Engineers (2017). "Ashrae Handbook- Hvac Applications").
- "UTA plant" wording means an equipment for air treatment in closed environments. Usually said equipment is composed of a thermal exchange battery for both cooling and heating, an air filter and a low prevalence suction/delivery fan.
- The wording "about" indicates a variation of the indicated value of +/- 2% of the indicated value.

Finally, except where specifically indicated, the percentages are intended to be% by weight.

### DETAILED DESCRIPTION OF THE INVENTION AND SOME OF ITS PREFERRED EMBODIMENTS

The method according to the invention preferably provides for the following steps in sequence:
- a step of making available appropriate raw materials;
- a step of supercritical solvothermal carbonization, wherein said raw materials are subjected to a carbonization process in a temperature range and supercritical pressure in an atmosphere composed of carbon dioxide (CO₂) in order to obtain a carbonized byproduct;
- a step of de-agglomeration, preferably by ultrasonics, wherein the carbonized byproduct is subject to a de-agglomeration process, preferably by ultrasonics in order to obtain a de-agglomerated byproduct;
- a crushing step, preferably through high frequency electromagnetic fields, wherein the de-agglomerated byproduct obtained from the de-agglomeration step is subjected to a crushing process, preferably through the action of a high frequency electromagnetic field in order to obtain a crushed byproduct;
- a step of subcritic dehydration, wherein the crushed byproduct obtained from the crushing step is subjected to a process of dehydration in subcritical conditions in order to obtain a dehydrated byproduct, which preferably corresponds to the final product of the method according to the invention.

### Step of making available the raw materials

- advantageously said step of making available appropriate raw materials provides for available at least one raw material of vegetable origin. Preferably said raw material of vegetable origin can include one or more of the following: *Vitis Vinifera, Olea Europea, Silybum Marianum, Camelia Sinensis, Cynara Scolymus, Curcuma Longa, Piper Nigrum, Allium Sativum, Saccarum Officinarum, Solanum Lycopersicum, Zingiber Officinale Roscoe, Triticum Dicoccum, Raphanus Sativus, Juniperus Cedrus, Pinus Strobus, Artemisia Judaica, Azadirachta Indica, Thujopsis Dolabrata, Thuja Plicata, Citrus Sinensis, Helianthus Annuus, Solanum Lycopersicum, Aristea Ecklonii, Chenopodium Ambrosioides, Diospyros Mespiliformis, Rosmarinum Officinalis, Elephantorrhiza Elephantina, Eucalyptus Camaldulensis, Gunnera Perpensa, Harpephyllum Caffrum, Hypericum Perforatum, Melianthus Comosus, Terminalia Sericea, Warburgia Salutaris, Macrocystis Pyrifera (L.)C.A.Agardh, Azolla Caroliniana Willd, Arthrospira platensis, Rosa Chinensis.* The vegetable material used is essentially represented by stems, leaves and roots, it is used fresh (or stored under control under controlled temperature) and does not undergo other pretreatments,
- properly, moreover, said step of making available appropriate raw materials provides to make available at least a catalyst configured to promote the formation of intermediate clusters. Appropriately said catalyst can be selected between one or more of the following: for the materials to be allocated to purely antibacterial applications the main components are: pyridinium hydrochloride, aluminum titanate, Calix (4) pyridine, copper selenide; the secondary components are: recombinases, polymerases and lisozyme. For the materials to be allocated to purely antiviral applications, the main components are: carboxylated triazole, copper selenide, recombinases and lisozyme; the secondary components are aluminum titanate, Calix (4) pyridine, pyridinium hydrochloride and polymerases,
- polymerases with a % between 5% and 25%, essentially polymerase L and deoxyribonucleic polymerase,
- Lisozyme with a % between 5% and 20%, essentially EC 3.5 (to hydrolyze carbon-nitrogen bonds), EC 3.7 (to hydrolyse carbon-carbon bonds),
- Calix [4] pyridine with a % between 5% and 15%, essentially Calix [4] arene-25,26,27,28-tethrol,
- recombinases with a % between 10% and 20%, essentially recombinase RAD51,
- aluminum titanate (Al₂TiO₅) with a % between 5% and 10%,
- carboxylated triazole with a % between 5% and 25%, essentially methyl 1,2,4-triazole-3-carboxylate,
- Pyridinium chlorochromate with a % between 5% and 25%,
- Copper selenide (SeCu and/or SeCu₂, preferably SeCu₂) with a % of 5% and 15%.

The catalyst mixture is prepared by weighing the different types of catalysts to be used according to the material to be produced. Based on the weight of the vegetable material used, the amount of catalyst can vary between a minimum of 15% to a maximum of 25% of the weight (+/-2%) compared to the vegetable matrices used.

Advantageously said starting raw materials can present a humidity between 35 and 65%.

Preferably, said starting raw materials can present a size between 1 mm and 10 mm. Suitably, if necessary, a preliminary step of chopping and/or mincing can be made in order to obtain raw materials with an adequate size and/or granulometry.
- The vegetable material is placed in solid and grossly shredded form if necessary. The ratio between vegetable material and supercritical fluid depends on the volume of the furnace, usually in a 250 cm³ vessel, 100 gr of vegetable material and about 118 gr of CO₂ are used.

### Supercritical solvothermal carbonization step

Suitably said starting raw materials made available during this step of making available can be subjected to a supercritic solvothermal carbonization step in order to obtain a carbonized byproduct.

Advantageously said step of supercritical solvothermal carbonization can be made in controlled atmosphere, preferably in a CO₂ atmosphere, and more preferably in a saturated CO₂ atmosphere in gaseous state.

Preferably, in said step of supercritical solvothermal carbonization, the starting raw materials, made available during the making available step, are heated at a temperature between 150°C and 250°C, preferably 195°C.

Preferably, in said step of supercritical solvothermal carbonization, the starting raw materials, made available during the making available step, are subjected to a pressure between 120 bar and 240 bar preferably about 160 bar.

Preferably at this step of supercritical solvothermal carbonization, it can last for a period between 2 hours and 8 hours, preferably 4 hours.

Advantageously said supercritic solvothermal carbonization step can be made within a vessel closed and separated from the external environment, preferably an autoclave.

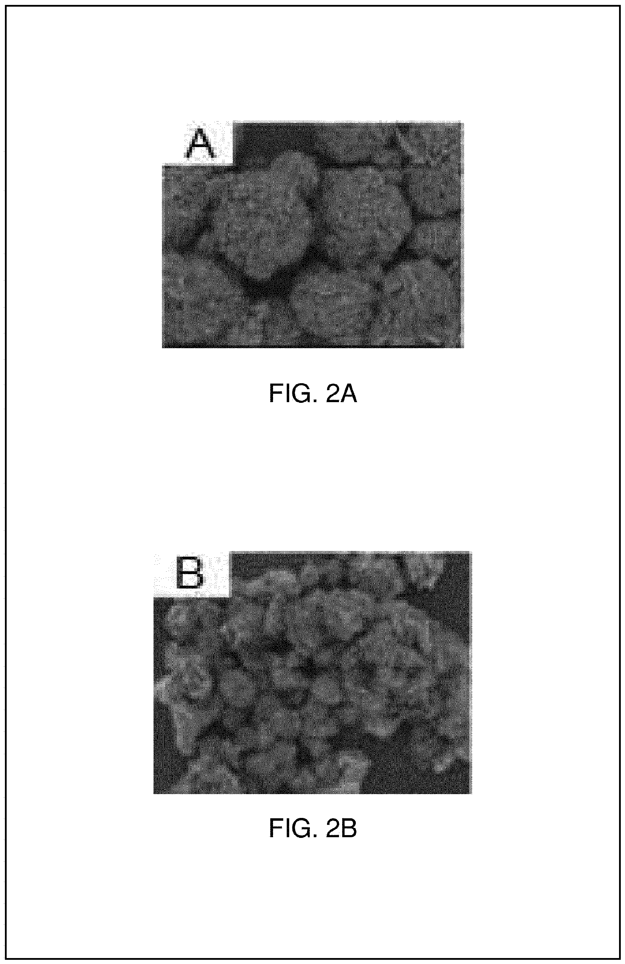

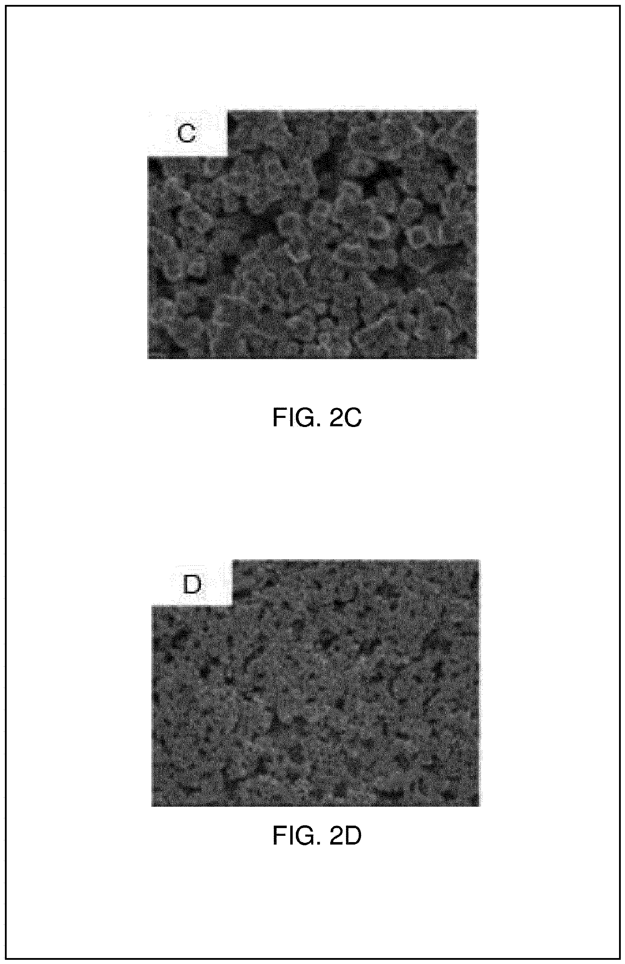

Appropriately at the end of the supercritical solvothermal carbonization step, a carbonized byproduct is obtained consisting of solid agglomerations (typically solid with an average humidity of 4-5%) which includes nano-particles of carbon-based material, the material obtained having a size of about a few dozen microns, for instance 30 µm (see fig. 2A).

### De-agglomeration step

Advantageously said de-agglomeration step may subject for the carbonized byproduct, obtained from the supercritical solvothermal carbonization step, to high frequency sound impulses in order to obtain a de-agglomerated byproduct.

The de-agglomeration step can be advantageously performed later, and preferably just later, compared to the supercritical solvothermal carbonization step (usually within 30 minutes from the carbonization step).

Appropriately during said de-agglomeration step, sound impulses with frequencies between 25 and 50 khz can be used.

Suitably the de-agglomation step can last between 20 minutes and 2 hours.

Preferably, at the beginning of the de-agglomeration step by ultrasonics, the mixture of vegetable and catalyst species has a size lower than a micron, for instance a size between 0.1 micron and 0.5 microns, and preferably about 0.2 µm (see Fig. 2b)

### Crushing step

The crushing step can be carried out subsequently, and preferably just later (usually within 1 h from the de-agglomeration step), compared to the de-agglomeration step. Suitably the crushing step can allow to obtain a crushed byproduct.

For crushing, a jaw crusher RetSch BB50 model is usually used.

Appropriately said crushing step can be configured to reduce the dimensions of said de-agglomerated byproduct up to a crushed byproduct, wherein nanoparticles have a typical size of some nm, preferably about 5 nm (see Fig. 2C).

Suitably during said crushing step said de-agglomerated byproduct can be subjected to electromagnetic fields that generate a magnetic flow density greater than 0.1 T, and preferably between 0.2 and 0.6 T, preferably 0.45 T.

Advantageously said electromagnetic fields can be substantially radio-waves, and preferably have a frequency between 5 and 10 GHz.

Suitably this crushing step can last between 40 and 120 minutes preferably 90 minutes.

### Dehydration step

A percentage of humidity always remains before the dehydration step, which is estimated in about 3-4%, therefore advantageously the subcritic dehydration step can be carried out subsequently, and preferably just later (usually within 30 minutes from the crushing step), compared to the crushing step.

Suitably said dehydration step can be carried out in order to transform said crushed byproduct into a dehydrated byproduct, which can basically correspond to the final product.

Advantageously during said dehydration step said crushed byproduct can be brought and, preferably maintained, at a temperature between 45 and 105°C, preferably 80°C.

Advantageously during said dehydration step said crushed byproduct can be brought and, preferably maintained, at a pressure between 70 and 90 bar, preferably 80 bars.

Advantageously said dehydration step can last for a time between 1 and 6 hours, and preferably 4 hours.

The dehydration step can advantageously lead to a further separation of the agglomerations of Nano-Cluster that make up the material. In fact, the dehydration step involves the loss of all the residual humidity that leads to a better "comminuting" aka separation of the nano cluster agglomerates.

### Final product

Appropriately the final product can have the following characteristics, which can be measured by the following techniques: HR-TEM (*High Resolution Transmission Electron Microscope*), XRD (*X-ray diffraction*) and FT-IR (*Fourier-Transform Infrared Spectroscopy*)*,* Raman Spectroscopy:
Dimensions: between 1 and 6 nanometers. Typically from 100 gr of vegetable material, about 40 g of finished material are obtained on average,
Composition: carbon between 98.8 and 99.8%, hydrogen between 0.1% and 0.7%, oxygen between 0.1%and 0.5%,
Relative humidity: 1-2%,
Morphology: number of OH hydroxyl groups between 50,000 and 250,000; Carbon-based nano-cluster in a number between 200,000 and 500,000, these values refer to the number of hydroxyl groups per gram of material analyzed with infrared spectrographs (IR) and the number of nanoclusters per gram of material analyzed using transmission electronic microscopy systems (TEM); using TEM, it's possible to directly view the nanoclusters and count their number by specific image analysis software.

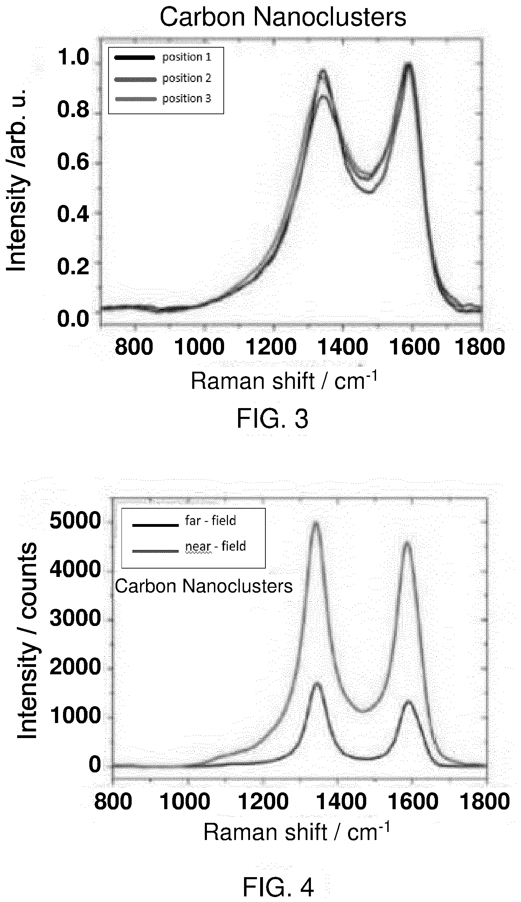

The presence of carbon atoms with sp² hybridization can be easily measured by means of Raman spectroscopy, in particular it is observed (see fig. 3) a peak around 1300 cm⁻¹ and a peak around 1600 cm⁻¹.

### Examples

For a better understanding of this invention, examples of implementation for illustrative and non-limiting purposes are reported below.

### Example 1, PREPARATION OF A NEW ANTIMICROBIAL MATERIAL AS A HYDROPHILIC BASE FOR MEDICAL SURGICAL AID FOR ANTIBACTERIAL TREATMENT OF HOSPITAL SHEETS

### - Supercritic Solvothermal Carbonization Step

Within a Ethos One type furnace from Milestones Inc. the starting raw materials are placed, which comprise 100 g of a mixture as follows: 50 g of grape seeds from *Vitis Vinifera* with an average diameter of 1 mm and having a 50% humidity, 30 g of *Olea Europea* nuts with an average diameter of 2 mm and having a 55% humidity and 5 g of *Piper Nigrum* berries with an average diameter of 5 mm and a humidity of 45%, 1 gr of L-Polimerase (Cas Number 577784-19-19 Sigma Aldrich), 2.5 gr of Calix [4] pirydine (Cas Number 74568-07-03 Sigma Aldrich) and 1.5 gr of Ricombinase RAD 51 (Cas Number 1290541-6 Sigma Aldrich), 5 grams of aluminum titanate (Cas number 12004-39-6 Sigma Aldrich) and 5 grams of Pyridinium Chlorochromate (Cas Number 26299-14-9 Merck).

The mixture is subjected to a 150°C temperature with a 120 bar pressure and saturation with supercritical carbon dioxide for a period of 120 minutes in order to obtain said carbonized byproduct.

### - De-agglomeration Step

Within an UIP500 HDT model ultrasonicator from Hielscher, the carbonized byproduct is introduced, and the latter is undergoing ultrasonic treatment with a 25 KHz frequency, for a 20 minutes period to obtain said de-agglomerate byproduct.

### - Crushing Step

The de-agglomerated byproduct is subjected to an electromagnetic field having an inductive power of 0.2 Tesla with a pulsed frequency of 5 GHz for a 50 minutes period to obtain the crushed byproduct.

### - Subcritic Dehydration Step

The crushed byproduct is dehydrated at a 45°C temperature at a 70 bar pressure for a period of 2 hours.

From the high pressure dehydration step, about 45 g of a dehydrated byproduct are obtained, which substantially corresponds to the final product of the method according to the invention.The powder materials obtained were analyzed with HR-TEM, XRD and FT-IR methodologies, and on average they have these main features on a quantity by weight of1 gr:
Dimensions: 3 nm,
Composition: carbon: 99.7%, hydrogen: 0.2%, oxygen: 0.1%,
Relative humidity 1%,
Number of hydroxy groups OH: 120,000, number of carbon-based nano-cluster: 250,000,
Presence of carbon atoms with sp² hybridization, as can be easily measured by Raman spectroscopy, in particular the presence of a peak around 1300 cm⁻¹ and a peak around 1600 cm⁻¹ is highlighted (see Fig.3).

The material obtained at the end of the different steps described above was subjected to testing of the antimicrobial activity in comparison with traditional antimicrobial additives such as Triclosan and Isotiazolone, used for the antibacterial treatment of cotton sheets for hospital use.

A 10% aliquot (4.5 gr) of the final product was diluted in 1.35 gr of DMSO (dimetylsolfoxyide) and with the solution obtained, the cotton fabrics have been soaked by manual splashing; the treated tissues were left to dry at room temperature and subjected to antibacterial activity evaluation tests.

In Table 1 that follows, are compared with methodology (ISO) 20645 and Japan Industrial Standards (JIS) Z 2801: 2000 the antibacterial properties of the preparation, according tothis invention, using the "pour plate" technique with *Escherichia coli* microorganisms (ATCC 25922) and *Bacillus Subtilis* (ATCC 6633) measuring the inhibition halos. Table 1 shows the antibacterial efficacy values of the cotton of the sheets treated with the different types of preparations measured by the diameter of the inhibitory halo (bactericidal area) for each tested sample.

**Table 1: observed antibacterial efficacy test by measuring the halo of bacterial inhibition for bacterial species Escherichia coli and Bacillus subtilis on the cotton matrix treated with the different compounds.**

| | Bacterial inhibition halo (diameter in mm) | |
|---|---|---|
| Bacterial species | *Escherichia Coli* | *Bacillus Subtilis* |
| Analyzed materials | | |
| Triclosan | 2.10 | 2.28 |
| Isotiazolone | 0.1 | 0.5 |
| Preparation according to the invention | 4.83 | 4.88 |

The table highlights a high antibacterial capacity of the preparation 1 according to the invention, against traditional antibacterial products currently widely used in the sector of medical surgical aids.

### Comparison example 1

A product was prepared on the basis of the indications of IT patent application 2018 0000 9526. The product had the following characteristics:
average dimensions about 50 nm; presence of hydroxyl groups OH not exceeding 500 and number of carbon nanoclusters not exceeding 25,000 per gram of material. Instead the material of the invention, prepared according to example 1, has average dimensions of about 5 nm, has a number of hydroxyl groups about 150,000 and a number of carbon nanoclusters not less than 200,000 per gram of material.

The antimicrobial activity vs bacterial and fungal strains commonly present in the dermatological sector has been compared, with a new comparative study between the material prepared following the cited patent application and the current product, evaluating the MIC *in vitro* (minimum inhibitory concentration expressed in mg/lit). The results, shown in Table 2, show a marked improvement of the antimicrobial capabilities of the current material vs the previous material, compared with the antibacterial and antifungal capabilties of the three traditional molecules most used in the dermatological sector for topical use.

**Table 2**

| Strain | Product of Patent IT 2018 | Product of the invention | Catechin | Inokithiol | Ketoconazole |
|---|---|---|---|---|---|
| *Propionibacterium Acnes* (Strain ATCC 6919) | 100 | 55 | 2560 | 64 | 210 |
| *Staphylococcus Epidermis* (Strain ATCC 12228) | 15 | 1 | 2560 | 0.5 | 5 |
| *Candida Albicans* (Strain NBRC 1594) | 12 | 3 | N.O. | 2 | 5 |

### Example 2 PREPARATION OF A NEW ANTIVIRAL MATERIAL AS A HYDROPHILIC BASE FOR MEDICAL SURGICAL AID FOR THE ANTIVIRAL TREATMENT OF AIR FILTERING MEDIA

### - Supercritic Solvothermal Carbonization Step

Within a Ethos One type furnace from Milestones Inc. the starting raw materials are placed, in particular 100 g of a mixture as follows: 50 g of *Raphanus Sativum* root with an average 5 mm diameter and having a 50% humidity, 30 g of *Thuja Plicata* root with an average 6 mm diameter and having a 55% humidity, 5 gr of Lisozyme EC 3.7 (Cas Number 9001-63-2 Merck), 5 gr of Ricombinase RAD 51 (Cas Number 1290541-46-6 Sigma Aldrich) and 10 gr of carboxylated triazole (Cas number 288-88-0 Merck).

This mixture is subjected to a 200°C temperature with a 200 bar pressure and saturation with supercritical carbon dioxide for a period of 180 minutes in order to obtain the carbonized byproduct.

### - De-agglomeration step

Within an UIP500 HDT model ultrasonicator from Hielscher, about 53 g of the carbonized byproduct obtained from the supercritical solvothermal carbonization step are placed and it is subjected to ultrasonics with a 30 kHz frequency, for 40 minutes in order to obtain the de-agglomerated byproduct.

### - Crushing step

The de-agglomerated byproduct is then subjected to an electromagnetic field having an inductive power of 0.3 Tesla with a pulsed 6 GHz frequency for a period of 90 minutes in order to obtain the crushed byproduct.

### - Subcritic dehydration step

The crushed byproduct is dehydrated at a 60°C temperature at a 80 bar pressure for a period of 4 hours in order to obtain the dehydrated byproduct, which substantially corresponds to the final product of the method according to the invention.

From the subcritic dehydration step, about 46 g of final product are obtained.

The powder materials obtained were analyzed with HR-TEM, XRD and FT-IR methodologies, and on average show these main features on a quantity by weight of 1 gr.
Dimensions 5 nm,
Composition: carbon: 99.8%, hydrogen: 0.1%, oxygen: 0.1%,
Relative humidity: 1%,
Number of hydroxyl groups OH: 50,000, number of carbon-based nano-clusters: 125,000,
Presence of carbon atoms with sp² hybridization, as can be easily measured by means of Raman spectroscopy, in particular the presence of a peak around 1300 cm⁻¹ is highlighted and a peak around 1600 cm⁻¹, and the band at 1300 cm⁻¹ is more intense than the 1300 cm⁻¹ band (see Fig. 4).

A 10% aliquot (4.6 gr) of the final product was diluted with a quantity of 1.15 g of DMSO (dimetylsolfoxyide) and with the solution obtained, filtering polyester medium were soaked which were subjected to testing for the evaluation of the antiviral activity towards the respiratory virus SARS COV2 strain B1.1.7 (Covid 19) and the respiratory virus A/H1N1 (seasonal influence 2022) to test the efficacy as a medical surgical aid for the antiviral treatment of filtering media for UTA devices

The viral strains were quantified using an ARN amplification method. The reverse transcription polymerase chain reaction system (RT-PCR) (Thermo Scientific^{™} Pikoreal^{™}) was used to amplify the virus RNA.

The overall antiviral effectiveness was determined by the analysis via PCR (Thermofisher^{™} Applied Biosystems^{™}).

In Table 3, the antiviral activity of the preparation, according to the invention, against the types of respiratory viruses tested in comparison with the most used surgical and medical aids for the disinfection of the filter media for UTA systems.

**Table 3: Value of the antiviral activity observed against the B1.1.7 Sars CoV2 viral strain and the A/H1N1 viral strain of the preparation according to the invention and the two most common virucidal agents used nowadays.**

| **Viral Strain** | **Contaminated Culture Medium** | **Preparation According to the invention** | **QUAT/s (Conc. 1.5%)** | **Sodium Hypochlorite (Cone. 1,5%)** |
|---|---|---|---|---|
| Virus Sars CoV2 (Strain B1.1.7) | 95 (HAU/ml) | 1.04 (HAU/ml) | 28.8 (HAU/ml) | 29.2 (HAU/ml) |
| Flu Virus A (Strain A/H1N1) | 114 (HAU/ml) | 1.02 (HAU/ml) | 20.8 (HAU/ml) | 20.1 (HAU/ml) |

The table shows a high antiviral capacity of the preparation according to the invention against the two currently best known viral strains with an average killing rate equal to about 99% against an average killing rate equal to about 70%.

The product prepared on the basis of the indications of the patent application IT 2018 0000 9526 did not show any antiviral capacity.

From what has been said it is clear how the method according to the invention allows to produce a material with excellent antibacterial and antiviral properties, starting from raw materials predominantly of natural origin.

## Claims

1. A method for the preparation of a material comprising, preferably in operative sequence, the following steps:
- a step of making at least one raw material available comprising at least one vegetable material chosen from among: *Vitis Vinifera, Olea Europea, Silybum Marianum, Camelia Sinensis, Cynara Scolymus, Curcuma Longa, Piper Nigrum, Allium Sativum, Saccarum Officinarum, Solanum Lycopersicum, Zingiber Officinale Roscoe, Triticum Dicoccum, Raphanus Sativus, Juniperus Cedrus, Pinus Strobus, Artemisia Judaica, Azadirachta Indica, Thujopsis Dolabrata, Thuja Plicata, Citrus Sinensis, Helianthus Annuus, Solanum Lycopersicum, Aristea Ecklonii, Chenopodium Ambrosioides, Diospyros Mespiliformis, Rosmarinum Officinalis, Elephantorrhiza Elephantina, Eucalyptus Camaldulensis, Gunnera Perpensa, Harpephyllum Caffrum, Hypericum Perforatum, Melianthus Comosus, Terminalia Sericea, Warburgia Salutaris, Macrocystis Pyrifera (L.)C.A.Agardh, Azolla Caroliniana Willd, Arthrospira platensis, Rosa Chinensis,* and at least one catalyst selected from: polymerase, lysozyme, Calix[4]pyridine, recombinase, Aluminum Titanate (Al₂TiO₅), Triazole Carboxylate, Pyridinium Chlorochromate, Copper Selenide;
- a supercritical solvothermal carbonization step, wherein said raw material is subjected to a carbonization process in a temperature range between 150°C and 250°C and a pressure between 120 bar and 240 bar in a supercritical CO₂ atmosphere in order to obtain a carbonized byproduct;
- a de-agglomeration step, wherein the carbonized byproduct is subjected to de-agglomeration, preferably by ultrasonication at frequencies between 25 and 50 kHz, in order to obtain a de-agglomerated byproduct;
- a crushing step, during which the de-agglomerated byproduct is subjected to a crushing process, preferably by the action of an electromagnetic field which generates a magnetic flux density higher than 0.1 T, more preferably at a frequency between 5 and 10 GHz;
- a subcritical dehydration step, during which the de-agglomerated byproduct is subjected to a dehydration process under subcritical temperature and pressure conditions with a temperature between 45 and 105°C and a pressure between 70 and 90 bar.

2. The method according to claim 1, **characterized in that** said catalysts comprise at least one of the following:
- Polymerase, preferably with a % by weight between 5% and 25%,
- lysozyme, preferably with a % by weight between 5% and 20%,
- Calix[4]pyridine, preferably with a % by weight between 5% and 15%
- Recombinase, preferably with a % by weight between 10% and 20%,
- Aluminum Titanate, preferably with a % by weight between 5% and 10%,
- Triazole Carboxylate, preferably with a % by weight between 5% and 25%,
- Pyridinium Chlorochromate, preferably with a % by weight between 5% and 25%,
- Copper selenide, preferably with a % by weight between 5% and 15%.

3. The method according to one or more of the preceding claims, **characterized in that** said vegetable materials are essentially stems, leaves and roots and are used fresh or vacuum-packed at a controlled temperature.

4. The method according to one or more of the preceding claims, **characterized in that** said raw materials:
- have a size between 1 mm and 10 mm;
- have a humidity between 35% and 65% by weight.

5. The method according to one or more of the preceding claims, **characterized in that** in said supercritical solvothermal carbonization step, said raw materials:
- are heated to a temperature between 150°C and 250°C, and
- are subjected to a pressure between 120 bar and 240 bar and
- this step lasts for a period between 2 hours and 8 hours.

6. The method according to one or more of the preceding claims, **characterized in that** in said de-agglomeration step said carbonized byproduct is subjected to sound waves with frequencies between 25 KHz and 50 KHz, and, preferably said de-agglomeration step lasts between 20 minutes and 2 hours.

7. The method according to one or more of the preceding claims, **characterized in that** at the end of said de-agglomeration step by ultrasonication the de-agglomerated byproduct has a size comprised between 0.1 micron and 0.5 micron.

8. The method according to one or more of the preceding claims, **characterized in that** in said crushing step:
- the magnetic induction power is between 0.2 and 0.6 Tesla, and
- the electromagnetic pulse frequency is between 5 and 10 GHz, and
- said crushing step lasts between 40 and 120 minutes.

9. The method according to one or more of the preceding claims, **characterized in that** in said subcritical dehydration step said crushed byproduct is subjected to:
- a temperature between 45°C and 105°C, and
- a pressure between 70 bar and 90 bar, and
- this subcritical dehydration step lasts between 1 and 6 hours.

10. A material for use in surgical medical devices and/or medical devices and/or in the veterinary field made by a method according to one or more of the preceding claims **characterized in that** it comprises nano-clusters having the following characteristics:
- Size: between 1 and 6 nanometers, and
- Composition: Carbon between 98.8 and 99.8% by weight, Hydrogen between 0.1% by weight and 0.7% by weight, Oxygen between 0.1% by weight, and 0.5% by weight, and
- Relative Humidity: 1-2%, and
- Morphology: number of OH hydroxyl groups in a number between 50,000 and 250,000; between 200,000 and 500,000 carbon-based nano-clusters, and with predominantly sp² hybridized carbon atoms.
